(19) Europäisches Patentamt
European Patent Office
Office européen des brevets

(11) Veröffentlichungsnummer: **0 035 711**
**B1**

(12) **EUROPÄISCHE PATENTSCHRIFT**

(45) Veröffentlichungstag der Patentschrift: **09.05.84**

(51) Int. Cl.³: **C 07 D 295/14**, C 07 C 121/66, C 07 D 243/08, A 61 K 31/50, A 61 K 31/55

(21) Anmeldenummer: **81101419.0**

(22) Anmeldetag: **27.02.81**

(54) **10-substituierte 5-Cyanmethylen-dibenzo(a,d)-cycloheptene, Verfahren zu ihrer Herstellung und diese enthaltende therapeutische Mittel.**

(30) Priorität: **08.03.80 DE 3009034**

(43) Veröffentlichungstag der Anmeldung: **16.09.81 Patentblatt 81/37**

(45) Bekanntmachung des Hinweises auf die Patenterteilung: **09.05.84 Patentblatt 84/19**

(84) Benannte Vertragsstaaten: **AT BE CH DE FR GB IT LI LU NL SE**

(56) Entgegenhaltungen:
**CA - A - 1 000 701**
**DE - A - 1 568 089**
**DE - A - 1 620 151**
**DE - A - 1 620 155**
**DE - A - 2 918 778**

**ARZNEIMITTELFORSCHUNG, band 25, Nr. 5, Veröffentlicht in 1975, Seiten 712-720 Aulenburg, DE. J. SCHMUTZ: "Neuroleptic piperazinyl-dibenzo-azepines"**

(73) Patentinhaber: **BASF Aktiengesellschaft, Carl-Bosch-Strasse 38, D-6700 Ludwigshafen (DE)**

(72) Erfinder: **Steiner, Gerd, Dr., Oberer Waldweg 1, D-6719 Kirchheim (DE)**
Erfinder: **Hofmann, Hans Peter, Dr., Untere Hart 12, D-6703 Limburgerhof (DE)**
Erfinder: **Kreiskott, Horst, Dr., Am Boehlig, D-6706 Wachenheim (DE)**
Erfinder: **Teschendorf, Hans-Jürgen, Dr., René-Bohn-Strasse 4, D-6700 Ludwigshafen (DE)**
Erfinder: **Lenke, Dieter, Dr., Kékuléplatz 1, D-6700 Ludwigshafen (DE)**

**EP 0 035 711 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft in 10-Stellung substituierte 5-Cyanmethylen-dibenzo[a,d]-cycloheptene, ihre reinen cis- und trans-Isomeren, ihre N-Oxide und pharmazeutisch verträglichen Säureadditionssalze, Verfahren zu ihrer Herstellung, diese enthaltende therapeutische Mittel und die Verbindungen zur Anwendung als Arzneimittel, insbesondere als Sedativa, Hypnotika oder Tranquilizer.

Es ist bekannt, dass tricyclische Ringsysteme mit einer Dibenzo-struktur mit zentralem heterocyclischen oder isocyclischen 6,7- oder 8-Ring, der gegebenenfalls einen basischen Seitenrest, wie beispielsweise einen N-Methylpiperazinrest aufweist, neuroleptische Wirkungen besitzen können. Solche Tricyclen sind beispielweise N-Methylpiperazin-Derivate von

Dibenzo[b,e][1,4]-diazepinen (Clozapine),
Dibenzo[b,f][1,4]-thiazepinen (Clotiapine),
Dibenzo[b,f][1,4]-oxazepinen (Loxapine) oder Morphantridinen (Perlapine), wie beispielsweise aus der Zusammenfassung von J. Schmutz in der Arzneimittelforschung 25, 712-720 (1975) sowie aus DE-B-1 620 151, DE-A-1 568 089, DE-A-1 620 155 und aus EP-81 101 422.4 (=EP-A-35714) hervorgeht.

In der Patentanmeldung EP-A-19 172 werden 6-substituierte 11-Alkylen-morphantridine mit wertvollen pharmakologischen Eigenschaften vorgeschlagen.

Gegenstand dieser Anmeldung sind Derivate mit einem abgewandelten Ringsystem, die ein unterschiedliches pharmakologisches Wirkprofil aufweisen.

Es wurde nun gefunden, dass 10-substituierte 5-Cyanmethylen-dibenzo[a,d]-cycloheptene der allgemeinen Formel (I)

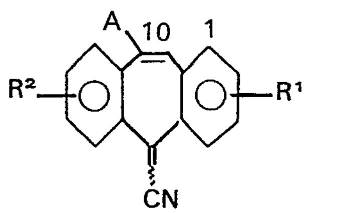

(I)

in der $R^1$ und $R^2$ Wasserstoffatome, Halogenatome, insbesondere Fluor, Chlor- oder Bromatome, Alkylreste mit 1 bis 3 C-Atomen oder Trifluormethyl bedeuten, und A einen Aminorest $-NR^4R^5$ bedeutet, in dem $R^4$ und $R^5$ Wasserstoffatome bedeuten oder $R^4$ und $R^5$ zusammen mit dem sie verbundenden Stickstoffatom einen 5- bis 7-gliedrigen gesättigten Ring bilden, der als weiteres Heteroatom ein Stickstoffatom enthält, wobei dieses gegebenenfalls durch einen Alkylrest mit 1 bis 3 C-Atomen oder einen Hydroxyalkylrest mit 2 bis 3 C-Atomen und gegebenenfalls zusätzlich durch Sauerstoff in Form eines N-Oxids substituiert ist,

ihre reinen cis- und trans-Isomeren und ihre physiologisch verträglichen Säureadditionssalze wertvolle pharmakologische Eigenschaften aufweisen.

Für die Reste $R^1$ und $R^2$ seien hervorgehoben Wasserstoff, Fluor, Chlor, Methyl und Trifluormethyl, wovon die Bedeutungen Wasserstoff und Chlor besonders bevorzugt sind.

Als Aminreste $-NR^4R^5$ für A, in denen $R^4$ und $R^5$ einen 5- bis 7-gliedrigen gesättigten Ring bilden, der als weiteres Heteroatom ein Stickstoffatom enthält, sind Piperazinyl- und Homopiperazinylreste zu nennen.

Besonders bevorzugte Reste $-NR^4R^5$ sind der 4-Methyl-piperazinyl-, 4-Methyl-4-oxy-piperazinyl-, 4-(2-Hydroxy)-äthyl-piperazinyl-, 4-Äthylpiperazinyl-, der N-Methyl-homopiperazinyl-Rest.

Es wird darauf hingewiesen, dass die erfindungsgemässen Verbindungen der Formel (I) als cis-trans-Isomere Ia und b vorliegen.

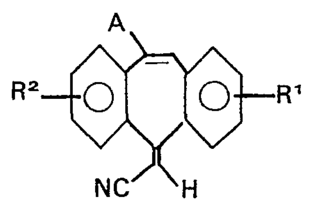

(Ia)

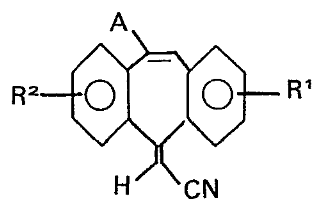

(Ib)

Gegebenenfalls können die cis-trans-Isomeren beispielsweise durch fraktionierte Kristallisation oder durch Säulenchromatographie getrennt werden. Die Zuordnung der einzelnen Isomeren erfolgt beispielsweise durch Röntgenstrukturanalyse, wie aus den Beispielen hervorgeht.

Aufgrund der oben genannten Bedeutungen sind die folgenden Verbindungen als besonders bevorzugt und wirksam zu nennen:

cis,trans-5-Cyanmethylen-10-(4-methyl-piperazin-1-yl)-dibenzo[a,d]-cyclohepten,
cis-5-Cyanmethylen-10-(4-methyl-piperazin-1-yl)-dibenzo[a,d]-cyclohepten,
trans-5-Cyanmethylen-10-(4-methyl-piperazin-1-yl)-dibenzo[a,d]-cyclohepten,
cis,trans-5-Cyanmethylen-10-(4-methyl-4-oxy-piperazin-1-yl)-dibenzo[a,d]-cyclohepten,
cis-5-Cyanmethylen-10-(4-methyl-4-oxy-piperazin-1-yl)-dibenzo[a,d]-cyclohepten,
trans-5-Cyanmethylen-10-(4-methyl-4-oxy-piperazin-1-yl)-dibenzo[a,d]-cyclohepten,
cis, trans-5-Cyanmethylen-2-chlor-10-(4-methyl-piperazin-1-yl)-dibenzo[a,d]-cyclohepten,
cis-5-Cyanmethylen-2-chlor-10-(4-methyl-piperazin-1-yl)-dibenzo[a,d]-cyclohepten,
trans-5-Cyanmethylen-2-chlor-10-(4-methyl-piperazin-1-yl)-dibenzo[a,d]-cyclohepten,
cis,trans-5-Cyanmethylen-10-(4-äthyl-piperazin-1-yl)-dibenzo[a,d]-cyclohepten,
cis-5-Cyanmethylen-10-(4-äthyl-piperazin-1-yl)-dibenzo-[a,d]-cyclohepten,
trans-5-Cyanmethylen-10-(4-äthyl-piperazin-1-yl)-dibenzo[a,d]-cyclohepten,

cis,trans-5-Cyanmethylen-10-(N'-methyl-homopiperazin-1-yl)-dibenzo[a,d]-cyclohepten, cis,trans-5-Cyanmethylen-10-(4-β'-hydroxyäthyl-piperazin-1-yl)-dibenzo[a,d]-cyclohepten.

Wie die Ausführungsbeispiele zeigen, kann im Einzelfall die Trennung in das cis- und trans-Isomere ohne allzu grossen Aufwand vorgenommen werden.

Die erfindungsgemässen Verbindungen der Formel (I) werden hergestellt, indem man eine Verbindung der Formel (II)

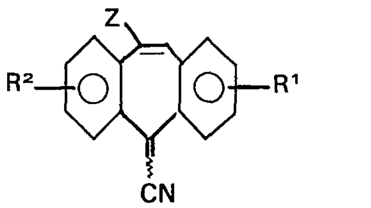
(II)

in der $R^1$ und $R^2$ die für Formel (I) angegebenen und bevorzugten Bedeutungen haben und Z Brom oder Chlor darstellt, mit einem Nukleophil AH, in dem A die für Formel (I) angegebenen Bedeutungen hat, umsetzt und gegebenenfalls die erhaltene Verbindung in das N-Oxid und/oder in das Säureadditionssalz einer physiologisch verträglichen Säure überführt.

Die Umsetzung erfolgt zweckmässig in Gegenwart einer überschüssigen Menge des der Formel AH entsprechenden Amins, das gegebenenfalls gleichzeitig als Lösungsmittel und gegebenenfalls als säurebindendes Mittel dient, in Gegenwart von 1 bis 2 Moläquivalent Kalium-tertiär-butylat. Gegebenenfalls kann in Gegenwart eines inerten Lösungsmittels, wie einem cyclischen gesättigten Äther, insbesondere Tetrahydrofuran oder Dioxan, von Benzol oder eines Benzolkohlenwasserstoffes, wie Xylol, Mesitylen oder Decahydronaphthalin gearbeitet werden. Die Umsetzung erfolgt in der Regel bei Temperaturen zwischen Raumtemperatur und 150°C und ist im allgemeinen innerhalb von 3 bis 10 Stunden beendet. Gegebenenfalls ist der Ausschluss des Sauerstoffs der Luft und das Arbeiten unter Inertgas, beispielsweise unter Stickstoff, von Vorteil.

Bei den Umsetzungen wird das Nukleophil AH vorteilhafterweise in mindestens 2-fachem molarem bis 20 molarem Überschuss verwendet.

Die Überführung einer Verbindung der Formel (I) in das N-Oxid erfolgt in üblicher Weise, zweckmässig mit wässrigem Wasserstoffperoxid (30 Gew.%) in äthanolischer Lösung. Die Überführung in das Säureadditionssalz einer physiologisch verträglichen Säure erfolgt ebenso in üblicher Weise.

Die Ausgangsverbindungen der Formel (II) werden durch Carbonyl-Olefinierung hergestellt, indem man ein 10-Brom-(Chlor)-dibenzo[a,d]-cyclohepten-5-on der Formel (III)

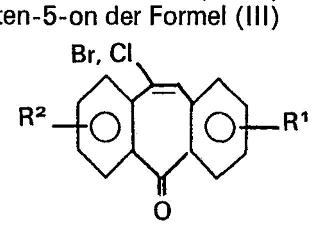
(III)

in der $R^1$ und $R^2$ die für Formel (I) angegebenen Bedeutungen haben, mit einem Phosphonat der Formel (IVa)

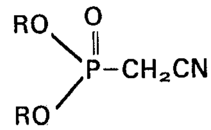
(IVa)

in der R einen Alkylrest mit 1 bis 3 C-Atomen bedeutet, unter den Bedingungen der Wittig-Horner-Reaktion in einem inerten Lösungsmittel, besonders bevorzugt Dimethylformamid, in Gegenwart von einem Moläquivalent einer Base, bevorzugt Natriumalkoholat, Natriumhydrid oder Natriumamid, bei Temperaturen von 20 bis 80°C umsetzt oder mit einem Phosphoniumsalz der Formel (IVb)

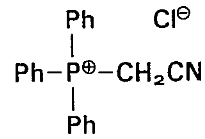
(IVb)

in der Ph für einen Phenylrest steht, unter den Bedingungen der klassischen Wittig-Reaktion in einem aprotischen organischen Lösungsmittel, insbesondere einem gesättigten aliphatischen oder gesättigten cyclischen Äther, wie Diäthyläther, Tetrahydrofuran oder Dioxan, oder vorzugsweise in Dimethylformamid, in Gegenwart von einem Moläquivalent einer Base, insbesondere einem Alkalialkoholat, vorzugsweise Natriummethylat oder Natriumäthylat, oder Natriumhydrid, Natriumamid oder einer metallorganischen Verbindung, wie Butyllithium, bei Temperaturen von 20 bis 100°C umsetzt.

Es soll an dieser Stelle betont werden, dass sich bei den obigen Umsetzungen die Reihenfolge von Carbonyl-Olefinierung und Einführung des nukleophilen Restes A in 10-Stellung mit gutem Erfolg umkehren lässt. Man geht in diesem Falle von dem entsprechenden 10-Brom-dibenzo[a,d]-cyclohepten-5-on aus und setzt mit dem Nukleophil AH zum 10-substituierten Dibenzo[a,d]-cyclohepten-Derivat der Formel (V) um.

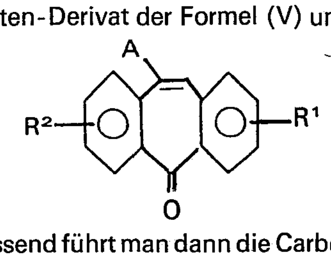
(V)

Anschliessend führt man dann die Carbonyl-Olefinierung wie oben beschrieben durch.

Die 10-Brom-(Chlor)-dibenzo[a,d]-cyclohepten-5-one der allgemeinen Formel (III) sind teilweise literaturbekannt (W. Treibs u. H.J. Klinkhammer, Ber. *84*, 671 (1951); H.L. Slates und N.L. Wendler, US-PS 3 297 763) oder können aus den entsprechenden Dibenzosuberon-Derivaten (vgl. E.L. Engelhardt et al., J. Med. Chem. *8*, 829 (1965)) durch zweifache Bromierung mit N-Brom-succinimid und nachfolgender Bromwasserstoff-Eliminierung mit methanolischer Kalilauge (vgl. E. Waldvogel et al., Helv. Chim. Acta *59*, 866 (1976)) hergestellt werden.

Neben den in den Beispielen aufgeführten Verbindungen werden folgende Verbindungen beispielhaft genannt:

cis,trans-8-Chlor-5-cyanmethylen-10-(4-methyl-piperazin-1-yl)-dibenzo[a,d]-cyclohepten,
cis,trans-2-Fluor-5-cyanmethylen-10-(4-methyl-piperazin-1-yl)-dibenzo[a,d]-cyclohepten,
cis,trans-8-Fluor-5-cyanmethylen-10-(4-methyl-piperazin-1-yl)-dibenzo[a,d]-cyclohepten,
cis,trans-2-Trifluormethyl-5-cyanmethylen-10-(4-methyl-piperazin-1-yl)-dibenzo[a,d]-cyclohepten,
cis,trans-8-Trifluormethyl-5-cyanmethylen-10-(4-methyl-piperazin-1-yl)-dibenzo[a,d]-cyclohepten.

Die erfindungsgemässen Verbindungen der Formel (I) werden in der Regel in Form von gelblichen bis gelben Kristallen erhalten und können durch Umkristallisation aus den üblichen organischen Lösungsmitteln, bevorzugt aus einem niederen Alkohol, wie Äthanol, umkristallisiert oder durch Säulenchromatographie gereinigt werden.

Falls erforderlich erfolgt eine Trennung in die einzelnen cis- und trans-Isomeren durch fraktionierte Kristallisation in einem chlorierten Kohlenwasserstoff, bevorzugt Methylenchlorid, einem niederen einwertigen Alkohol, bevorzugt Methanol oder Äthanol, oder einem gesättigten cycloaliphatischen Kohlenwasserstoff, bevorzugt Cyclohexan, oder durch Säulenchromatographie, insbesondere an Kieselgel, mit Methylenchlorid oder einer Mischung aus Methylenchlorid und Methanol im Verhältnis 99:1 bis 85:15 Volumenteilen.

Die freien 10-substituierten 5-Cyanmethylen-dibenzo[a,d]-cycloheptene der Formel (I) können in üblicher Weise in das Säureadditionssalz einer pharmakologisch verträglichen Säure überführt werden, vorzugsweise durch Versetzen einer Lösung mit einem Äquivalent der entsprechenden Säure. Als übliche physiologisch verträgliche anorganische Säuren kommen beispielsweise in Betracht Salzsäure, Bromwasserstoffsäure, Phosphorsäure oder Schwefelsäure und als organische Säuren beispielsweise Oxalsäure, Maleinsäure, Fumarsäure, Milchsäure, Weinsäure, Äpfelsäure, Zitronensäure, Salicylsäure, Adipinsäure oder Benzoesäure oder können aus Fortschritte der Arzneimittelforschung Band *10*, Seiten 224 bis 225, Birkhäuser Verlag, Basel und Stuttgart, 1966, entnommen werden.

Die erfindungsgemässen Verbindungen weisen wertvolle pharmakologische Eigenschaften auf. Sie können als Sedativa, Hypnotika, Tranquilizer, Neuroleptika oder Antidepressiva Verwendung finden. Mehrere der genannten Wirkungsqualitäten können kombiniert bei einer erfindungsgemässen Verbindung auftreten. In manchen Fällen kann das einzelne reine Isomere nach erfolgter Isomerentrennung eine Wirkung bevorzugt aufweisen.

Nach den Ergebnissen der durchgeführten pharmakologischen Experimente eignen sich die erfindungsgemässen Substanzen in Anbetracht ihrer sedativen-tranquillisierenden, muskelrelaxierenden und antimonaminergen Wirkung insbesondere als Sedativa, Hypnotika, Minor- oder Major-Tranquilizer.

Zur Wirkungsanalyse fanden folgende Methoden Verwendung:

1. *Sedative Wirkung*

4 bis 8 Gruppen von jeweils 3 weiblichen NMRI-Mäusen erhalten die Substanzen oral appliziert. Die photoelektrische Bestimmung der durch eine neue Umgebung induzierten Orientierungshypermotilität erfolgt 30 min nach der Applikation der Substanzen für eine Dauer von 30 min.

Als $ED_{50}$ wird die Dosis bestimmt, welche im Vergleich zu Placebo-behandelten Kontrolltieren eine Abnahme der Orientierungshypermotilität um 50% bewirkt.

2. *Muskelrelaxierende Wirkung*

Die Messung beruht auf der Quantifizierung des tonischen Dehnungsreflexes am Gastrocnemius des Kaninchen (Teschendorf et al., Arch. Pharmacol. exp. Path. *266*, 462, 1970). Das Kaninchen wird in einer speziellen Apparatur fixiert, die es erlaubt, die Pfote im Sprunggelenk in definierter und reproduzierbarer Weise zu beugen. Durch die Beugung wird in der Wadenmuskulatur ein tonischer Dehnungsreflex ausgelöst. Die elektrische Aktivität des Muskels während der Kontraktion wird registriert und die Einzelimpulse gezählt. Die Dehnung (Dauer 5 s) wird in Minutenabständen wiederholt. Nach Erreichen einer konstanten Impulszahl (Kontrollwert) wird die Testsubstanz intravenös appliziert. Die Impulszahlen nach Applikation werden auf die Vorwerte bezogen.

Tierzahl pro untersuchter Dosis 4 bis 6. Die $ED_{50}$ ist die Dosis, die die Muskelaktivität – bezogen auf den Vorwert – um die Hälfte reduziert.

3. *Antitryptamin-Wirkung*

Tryptamin-DCl (16 mg/kg i.v.) ruft an Ratten regelmässig folgende Symptome hervor: klonische Vorderpfotenbewegungen, Buckelbildung und Retropulsion sowie Kieferbewegungen (Tedeschi et al., J. Pharmacol. Exp. Ther. *126*, 223, 1959).

Die Prüfsubstanzen werden 30 min vor Tryptamin intraperitoneal verabfolgt. Kriterium für einen Substanzeffekt ist das Ausbleiben der Vorderpfotenbewegungen innerhalb eines 5-minütigen Beobachtungszeitraums nach Tryptamininjektion.

Als mittlere Hemmdosis ($ED_{50}$) wird mittels Probitanalyse die Dosis ermittelt, die das Symptom bei der Hälfte der Tiere verhindert.

4. *Anticholinerge Wirkung*

Gruppen von 10 weiblichen NMRI-Mäusen erhalten Physostigmin in einer letalen Dosis (0,825 mg/kg) subcutan. Die Prüfsubstanzen werden 30 min vor der Physostigminapplikation oral appliziert.

Als $ED_{50}$ wird die Substanzdosis bestimmt, welche 50% der Tiere vor dem Tod durch Physostigmin schützt.

5. *Akute Toxizität*

Gruppen von 5 bis 10 weiblichen NMRI-Mäusen erhalten die Substanzen i.p. Als $LD_{50}$ wird die Dosis ermittelt, nach welcher 50% der behandelten Tiere sterben.

In diesen Experimenten (vgl. Tabelle) wurden auffallend starke sedativ-hypnotische Wirkungen bei den erfindungsgemässen Substanzen nachgewiesen. Die Vergleichsverbindungen Clozapin und Perlapin werden mindestens erreicht, teilweise erheblich — bis zu 10-fach — übertroffen. Ferner weisen die Verbindungen stark ausgeprägte muskelrelaxierende Eigenschaften auf. Hierin sind sie den Referenzsubstanzen bis zum Faktor 100 überlegen. Diese Wirkungsqualitäten zeigen cis-trans-Gemische, besonders ausgeprägt aber die jeweiligen cis-Verbindungen der Beispiele 1a und 4a.

Als Parameter für eine neuroleptische Qualität kann eine antimonaminerge Wirkung — hier gemessen am Tryptaminantagonismus — gewertet werden. Auch in dieser Versuchsanordnung fanden sich deutliche Effekte, die zum Teil etwas geringer als bei den Referenzsubstanzen ausgeprägt sind, zum anderen aber bei der Verbindung Beispiel 4a deutlich stärker hervortreten.

Im Gegensatz zu Clozapin besitzen die neuen Verbindungen — wie der Antiphysostigmintest an der Maus zeigt — keine anticholinergen Eigenschaften, woraus auf geringere periphere Nebenwirkungen bei therapeutischer Anwendung zu schliessen ist.

Auf Grund der pharmakologischen Befunde können die erfindungsgemässen Verbindungen in entsprechenden galenischen Zubereitungen insbesondere als Sedativa, Hypnotika, Minor- bzw. Major-Tranquilizer verwendet werden.

*Tabelle*

| Beispiel Nr. | Sedative Wirkung | | Muskelrelaxation | | Antitryptamin-Wirkung | | Anticholinerge Wirkung $ED_{50}$ | Toxizität $LD_{50}$ |
|---|---|---|---|---|---|---|---|---|
| | $ED_{50}$ | R. W.[1] | $ED_{50}$ | R. W. | $ED_{50}$ | R. W. | | |
| 1 | 0,96 | 4,94 | 0,0064 | 7,19 | | | >10 | 324 |
| 1a | 0,80 | 5,92 | 0,00085 | 54,12 | 8,6 | 0,58 | >10 | |
| 2 | 2,0 | 2,37 | 0,0046 | 10,00 | | | >21,5 | |
| 2a | 0,44 | 10,77 | 0,0046 | 10,00 | 0,7 | 7,14 | >21,5 | |
| 3 | 1,38 | 3,43 | 0,013 | 3,54 | 18,6 | 0,27 | >21,5 | 287 |
| 3a | 1,0 | 4,74 | 0,01 | 4,60 | 11,2 | 0,45 | >21,5 | >100 |
| 5 | 0,97 | 4,89 | 0,012 | 3,54 | | | >21,5 | >100 |
| 6 | 1,4 | 3,39 | 0,1 | 0,46 | 12,5 | 0,40 | >21,5 | >100 |
| Clozapin | 4,74 | 1,00 | 0,046 | 1,00 | 5,0 | 1,00 | 14,1 | 215 |
| Perlapin | 2,01 | 2,36 | 0,1 | 0,46 | 4,1 | 1,22 | >21,5 | 215 |

[1] R. W. = relative Wirksamkeit

Die Erfindung betrifft dementsprechend auch ein therapeutisches Mittel, gekennzeichnet durch einen Gehalt an einer Verbindung der Formel (I) oder deren pharmakologisch verträgliches Säureadditionssalz als Wirkstoff neben üblichen Träger- und Verdünnungsmitteln.

Therapeutische Mittel mit üblichen Trägerstoffen oder Verdünnungsmitteln und den üblicherweise verwendeten technischen Hilfsstoffen können entsprechend der gewünschten Applikationsart und mit einer zur Anwendung geeigneten Dosiereinheit in an sich üblicher Weise hergestellt werden. Als Einzeldosen beim Menschen kommen 10 bis 100 mg in Betracht.

Die neuen Verbindungen können in den üblichen galenischen Applikationsformen, fest oder flüssig, angewendet werden, wie Tabletten, Kapseln, Pulver, Granulate, Dragees oder Lösungen. Diese werden in üblicher Weise hergestellt. Die Wirkstoffe können dabei mit den gebräuchlichen galenischen Hilfsmitteln, wie Talkum, Gummi arabicum, Saccharose, Lactose, Getreide- oder Maisstärke, Kartoffelmehl, Magnesiumstearat, Alginaten, Gummi tragacanth, Carraghenate, Polyvinylalkohol, Polyvinylpyrrolidon, wässrigen oder nicht wässrigen Trägern, Netzmitteln, Dispergiermitteln, Emulgatoren und/oder Konservierungsmitteln verarbeitet werden (vgl. L.G. Goodman, A. Gilman, The Pharmacological Basis of Therapeutics). Die so erhaltenen Präparate enthalten den Wirkstoff normalerweise in einer Menge von 0,001 und 99 Gew.%.

Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen, Suspensionen oder Depotformen. Es kommen auch parenterale Zubereitungen, wie Injektionslösungen, in Betracht. Weiterhin seien als Zubereitungen beispielsweise auch Suppositorien genannt.

Entsprechende Tabletten können beispielsweise durch Mischen des Wirkstoffs mit bekannten Hilfsstoffen, beispielsweise inerten Verdünnungsmitteln, wie Dextrose, Zucker, Sorbit, Mannit, Polyvinylpyrrolidon, Calciumcarbonat, Calciumphosphat oder Milchzucker, Sprengmitteln wie Maisstärke oder Alginsäure, Bindemitteln wie Stärke oder Gelatine, Gleitmitteln wie Magnesiumstearat oder Talk und/oder Mitteln zur Erzielung eines Depoteffektes, wie Carboxypolymethylen, Carboxymethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, biespielsweise Kollidon oder Schellack, Gummi arabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit den erfindungsgemässen Wirkstoffen können zusätzlich geschmacksverbessernde Mittel, wie Vanilin oder Orangenextrakt, enthalten. Sie können ausserdem Suspendierstoffe, die Natriumcarboxymethylcellulose, oder Konservierungsstoffe, wie p-Hydroxy-benzoate, enthalten. Wirkstoffe enthaltende Kapseln können beispielsweise hergestellt werden, indem man den Wirkstoff mit einem inerten Träger wie Milchzucker oder Sorbit mischt und in Gelatinekapseln einkapselt. Geeignete Suppositorien lassen sich beispielsweise durch Vermischen mit dafür vorgesehenen Trägermitteln, wie Neutralfetten oder Polyäthylenglykol bzw. deren Derivaten herstellen.

Die folgenden Beispiele dienen zur Erläuterung der Erfindung: Die angegebenen Schmelzpunkte der cis-trans-Isomerengemische können je nach dem cis-trans-Isomerenverhältnis etwas variieren.

*Beispiel 1:*

*cis- und trans-5-Cyanmethylen-10-(4-methylpiperazin-1-yl)-dibenzo[a,d]-cyclohepten*

20,0 g (65 mM) 10-Brom-5-cyanmethylen-dibenzo[a,d]-cyclohepten (cis,trans-Isomerengemisch) werden in 80 ml N-Methylpiperazin suspendiert. Unter Rühren gibt man portionsweise 8,6 g (84 mM) Kaliumtertiärbutylat hinzu und lässt das Reaktionsgemisch 10 bis 20 Std. bei Raumtemperatur unter Stickstoff rühren. Anschliessend giesst man auf Eiswasser und saugt das gelbliche Rohprodukt ab. Reinigung durch Umkristallisieren aus Äthanol oder durch Säulenchromatographie (Kieselgel, Methylenchlorid/Methanol 95/5). Man isoliert 17 g (80%) Produkt als cis,trans-Isomerengemisch, Schmp. 92-97° C.

Zur Trennung der cis, trans-Isomeren digeriert man das Isomerengemisch in ca. 80 ml siedendem Methanol und saugt die unlöslichen Anteile in der Hitze ab. Nach dem Nachwaschen mit wenig Methanol erhält man 3,2 g gelbe Festkörper, die nach Ausweis des Dünnschichtchromatogramms (Kieselgel, Laufmittel Toluol/Methanol 85/15) vornehmlich aus dem unpolaren cis-Isomeren a bestehen.

Die nach einigen Stunden kristallisierte 2. Fraktion besteht häufig aus dem Isomerengemisch. Danach kristallisiert jedoch aus der verbleibenden Mutterlauge 2,0-2,5 g des stark angereicherten polaren trans-Isomeren b, das abgesaugt und mit wenig Methanol nachgewaschen wird.

Durch Einengen der Mutterlauge, Zugabe des oben erhaltenen Isomerengemisches, nochmaligem Aufnehmen in 50 ml siedendem Methanol und mehrfachem Wiederholen der obigen Operationen erhält man weitere Fraktionen der stark angereicherten geometrischen Isomeren, die anschliessend noch aus Äthanol umkristallisiert werden. cis-Isomeres 1a: gelbe Kristalle mit Schmp. 199 bis 200, trans-Isomeres 1b: gelbe Kristalle mit Schmp. 189-191° C.

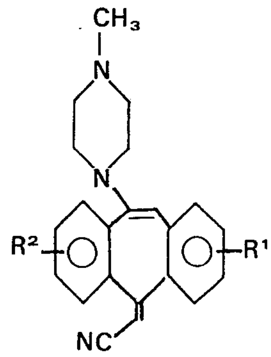
(a)

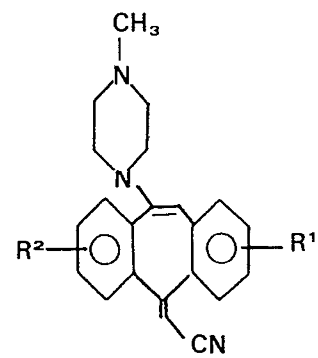
(b)

*Herstellung des Vorproduktes*

*cis,trans-10-Brom-5-cyanmethylen-dibenzo-[a,d]-cyclohepten*

Zur Herstellung des Vorproduktes 10-Brom-5-cyanmethylen-dibenzo[a,d]-cyclohepten führt man eine Carbonyl-Olefinierung des 10-Brom-dibenzo[a,d]-cyclohepten-5-ons mit Hilfe der Wittig-Horner-Reaktion oder über die klassische Wittig-Synthese durch:

30,0 g (105 mM) 10-Brom-dibenzo[a,d]-cyclohepten-5-on werden in 300 ml Dimethylformamid unter Erwärmen gelöst und unter Stickstoff gerührt. Man lässt dann gleichzeitig 31,8 g (180 mM) Diäthyl-cyanmethyl-phosphonat und 31,5 g (180 mM) Natriummethylat (30%) gelöst in 100 ml Dimethylformamid langsam zutropfen (Farbzunahme und Temperaturerhöhung zeigen den Beginn der Wittig-Reaktion an). Nach 18-stündigem Nachrühren bei Raumtemperatur giesst man das Reaktionsprodukt auf Eiswasser und saugt die ausfallenden Festkörper ab. Nach gutem Nachwaschen mit Wasser wird das Rohprodukt getrocknet und aus Äthanol umkristallisiert. Ausbeute: 28,5 g (88%) farblose Kristalle mit Schmp. 197-200° C.

Klassisches Wittig-Verfahren: Man legt Triphenyl-cyanomethyl-phosphonium-chlorid in Dimethylformamid vor, lässt anschliessend 1 Moläquivalent einer 30%igen Natriummethylat-Lösung zutropfen oder versetzt mit 1 Moläquivalent Natriumhydrid und fügt zuletzt 1 Moläquivalent einer Lösung von 10-Brom-dibenzo[a,d]-cyclohepten-5-on in Dimethylformamid hinzu. Man lässt das Reaktionsgemisch 5 bis 8 Stunden bei 50 bis 80° C nachrühren, giesst anschliessend auf Eiswasser und extrahiert mehrmals mit Methylenchlorid. Nach dem Trocknen und Einengen der organischen Phase kristallisiert man das

Rohprodukt aus Äthanol um. Ausbeute: 82% farblose Kristalle vom Schmp. 198-201°C.

*Herstellung des Vorproduktes*

*10-Brom-dibenzo[a,d]-cyclohepten-5-one*

Die als Ausgangsmaterialien für die Wittig-Reaktionen eingesetzten 10-Brom-dibenzo[a,d]-cyclohepten-5-one der allgemeinen Formel (III) werden entweder nach W. Treibs und J.J. Klinkhammer, Ber. *84*, 671 (1951) oder nach der Vorschrift von E. Waldvogel et al., Helv. Chim. Acta *59*, 866 (1976) hergestellt, z.B.:

a) 2-Chlor-dibenzosuberon: E.L. Engelhardt et al., J. Med. Chem. *8*, 829 (1965)

b) 2-Chlor-10-brom-dibenzo[a,d]-cyclohepten-5-on: Herstellung nach E. Waldvogel, Helv. Chim. Acta *59*, 866 (1976) durch zweifache Bromierung mit N-Brom-succinimid und anschliessender Bromwasserstoff-Eliminierung: Schmp. 127-129°C.

*Beispiel 2:*

*cis- und trans-2-Chlor-5-cyanmethylen-10-(4-methyl-piperazin-1-yl)-dibenzo[a,d]-cyclohepten*

Synthese analog Beispiel 1, unter Verwendung der entsprechenden in 2-Stellung chlorierten Derivate.

Man isoliert 84% 2-Chlor-5-cyanmethylen-10-(4-methyl-piperazin-1-yl)-dibenzo[a,d]-cyclohepten als cis,trans-Isomerengemisch mit dem Schmp. 196-198°C.

Zur Trennung der cis,trans-Isomeren digeriert man das Isomerengemisch in wenig siedendem Äthanol und saugt die unlöslichen Anteile in der Hitze ab. Nach dem Nachwaschen mit wenig Äthanol erhält man gelbe Festkörper, die nach Ausweis des Dünnschichtchromatogramms (Kieselgel, Laufmittel Toluol/Methanol 85/15) vornehmlich aus dem unpolaren cis-Isomeren a bestehen.

Die nach einigen Stunden kristallisierte Fraktion besteht aus dem stark angereicherten polaren trans-Isomeren b, das abgesaugt und mit wenig Äthanol nachgewaschen wird.

Durch mehrfaches Wiederholen der obigen Operationen erhält man weitere Fraktionen der stark angereicherten geometrischen Isomeren, die anschliessend noch aus Äthanol umkristallisiert werden. cis-Isomeres 4a: gelbe Kristalle mit Schmp. 227-229°C, trans-Isomeres 4b: gelbe Kristalle mit Schmp. 223-225°C.

Die bei dem Isomeren a durchgeführte Röntgenstrukturanalyse beweist die 2-Stellung des Chloratoms und ausserdem die cis-Stellung der Cyanmethylen-Gruppierung in Bezug auf den Piperazin-Ring.

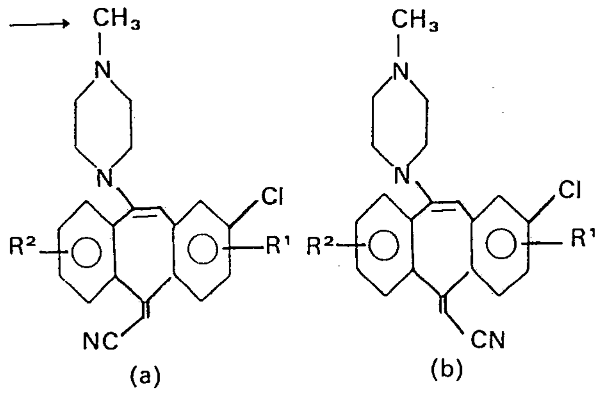

(a) (b)

*Beispiel 3:*

*cis,trans-5-Cyanmethylen-10-(4-äthyl-piperazin-1-yl)-dibenzo[a,d]-cyclohepten*

Synthese analog Beispiel 1 unter Einsatz von N-Äthyl-piperazin. Nach Umkristallisieren aus Methanol: gelbe Kristalle mit Schmp. 153-158°C.

Durch fraktionierte Kristallisation aus Methanol isoliert man die unpolare (Dünnschicht, Kieselgel, Laufmittel Toluol/Methanol 85/15) cis-Verbindung 5a mit Schmp. 164-166°C sowie das polare trans-Isomere 5b mit Schmp. 161-162°C in Form von gelben Nadeln.

*Beispiel 4:*

*cis,trans-5-Cyanmethylen-10-(N′-methyl-homopiperazin-1-yl)-dibenzo[a,d]-cyclohepten·½$H_2O$*

Synthese analog Beispiel 1 unter Einsatz von N-Methylhomopiperazin. Nach Reinigung durch Säulenchromatographie (Kieselgel, Methylenchlorid/Methanol 95/5): gelbe Kristalle mit Schmp. 80-86°C.

*Beispiel 5:*

*cis,trans-5-Cyanmethylen-10-(N-β-hydroxyäthyl-piperazin-1-yl)-dibenzo[a,d]-cyclohepten·½$H_2O$*

Synthese analog Beispiel 1 unter Einsatz von N-β-Hydroxyäthyl-piperazin und Erhöhung der Nachrührtemperatur auf 50°C während 3 Stunden. Nach Reinigung durch Säulenchromatographie (Kieselgel, Methylenchlorid/Methanol 95/5): gelbe Kristalle mit Schmp. 92-99°C.

*Beispiel 6:*

*cis,trans-5-Cyanmethylen-10-(4-methyl-4-oxy-piperazin-1-yl)-dibenzo[a,d]-cyclohepten·2½ $H_2O$*

3,0 g (9,2 mM) cis,trans-5-Cyanmethylen-10-(4-methylpiperazin-1-yl)-dibenzo[a,d]-cyclohepten (Beispiel 1) werden in 100 ml heissem Äthanol gelöst und mit 5 ml 30%igem Wasserstoffperoxid versetzt. Nach 5-stündigem Rückflusskochen zerstört man das überschüssige Wasserstoffperoxid mit Hilfe eines kleinen Platinblechs, das in das Reaktionsgemisch geworfen wird, durch 2stündiges Rückflusskochen. Nach Filtrieren engt man das Reaktionsgemisch ein und reinigt das erhaltene N-Oxid durch Säulenchromatographie (Kieselgel, Laufmittel Methylenchlorid/Methanol 95/5). Man isoliert 2,2 g (70%) gelbe Kristalle mit Schmp. 134-138°C.

*Beispiel 7:*

*Umkehrung der Reaktionsfolge von Carbonylolefinierung und Einführung des nukleophilen Restes A*

a) 5,0 g (17,5 mM) 10-Brom-dibenzo[a,d]-cyclohepten-5-on werden in 30 ml N-Methylpiperazin suspendiert. Unter Rühren gibt man portionsweise 4,8 g (47 mM) Kaliumtertiärbutylat hinzu und lässt das Reaktionsgemisch 6 Stunden bei 110°C unter Stickstoff rühren. Anschliessend destilliert man das überschüssige Methylpiperazin im Vakuum ab und verteilt den Rückstand zwischen Wasser und Methylenchlorid. Nach dreimaligem Waschen der organischen Phase mit Wasser und Trocknen isoliert man nach Einengen 4,9 g (92%) 10-(N-Methylpiperazin-1-yl)-dibenzo[a,d]-cyclohepten-5-on als Öl, das für die weitere Umsetzung genügend rein ist.

b) 3,1 g (10,2 mM) 10-(N-Methyl-piperazin-1-yl)-dibenzo[a,d]-cyclohepten-5-on werden in 30 ml Dimethylformamid gelöst und unter Stickstoff gerührt. Man lässt dann gleichzeitig 2,48 g (14 mM) Diäthylcyanmethyl-phosphonat und 2,45 g (14 mM) Natriummethylat (30%) gelöst in 10 ml Dimethylformamid langsam zutropfen (Farbzunahme und Temperaturerhöhung zeigen den Beginn der Wittig-Reaktion an). Nach 12-stündigem Nachrühren bei Raumtemperatur giesst man das Reaktionsprodukt auf Eiswasser und saugt die ausfallenden Festkörper ab. Nach Säulenchromatographie (Kieselgel, Methylenchlorid/Methanol 95/5) isoliert man das cis,trans-5-Cyanmethylen-10-(4-methyl-piperazin-1-yl)-dibenzo[a,d]-cyclohepten in 67% Ausbeute mit Schmp. 91-95°C.

*Beispiel 8:*

*cis,trans-5-Cyanmethylen-10-(piperazin-1-yl)-dibenzo[a,d]-cyclohepten·$H_2O$*

Synthese analog Beispiel 1 unter Verwendung von Dimethylformamid als Lösungsmittel und Einsatz von 5 Moläquivalenten Piperazin. Nach Reinigung durch Säulenchromatographie: gelbe Kristalle in 75% Ausbeute mit Schmp. 159-162°C.

Pharmazeutische Zubereitungen, die in üblicher Weise hergestellt werden:

*Beispiele für Tabletten:*

| | |
|---|---|
| 1. Ein Wirkstoff der Formel (I) | 5 mg |
| Lactose | 200 mg |
| Methylcellulose | 15 mg |
| Maisstärke | 50 mg |
| Talkum | 11 mg |
| Magnesiumstearat | 4 mg |

| | |
|---|---|
| 2. Ein Wirkstoff der Formel (I) | 20 mg |
| Lactose | 178 mg |
| Avicel | 80 mg |
| Polywachs 6000 | 20 mg |
| Magnesiumstearat | 2 mg |

| | |
|---|---|
| 3. Ein Wirkstoff der Formel (I) | 50 mg |
| Polyvinylpyrrolidon (mittl. M.G. 25 000) | 170 mg |
| Polyäthylenglykol (mittl. M.G. 4000) | 14 mg |
| Hydroxypropylmethylcellulose | 40 mg |
| Talkum | 4 mg |
| Magnesiumstearat | 2 mg |

Der Wirkstoff wird mit Polyvinylpyrrolidon in 10%iger wässriger Lösung befeuchtet, durch ein Sieb mit der lichten Maschenweite von 1,0 mm getrieben und bei 50°C getrocknet. Dieses Granulat wird mit Polyäthylenglykol (mittl. M.G. 4000), Hydroxypropylmethylcellulose, Talkum und Magnesiumstearat vermischt und zu Tabletten à 280 mg verpresst.

*4. Beispiel für Dragees*

| | |
|---|---|
| Ein Wirkstoff der Formel (I) | 60 mg |
| Lactose | 90 mg |
| Maisstärke | 60 mg |
| Polyvinylpyrrolidon | 6 mg |
| Magnesiumstearat | 1 mg |

Die Mischung der Wirkstoffsubstanz mit Lactose und Maisstärke wird mit einer 8%igen wässrigen Lösung des Polyvinylpyrrolidons durch ein Sieb (1,5 mm) granuliert, bei 50°C getrocknet und nochmals durch ein 1,0 mm Sieb gerieben. Das so erhaltene Granulat wird mit Magnesiumstearat gemischt und zu Drageekernen verpresst. Die erhaltenen Drageekerne werden in üblicher Weise mit einer Hülle überzogen, die im wesentlichen aus Zucker und Talkum besteht.

*5. Kapselformulierung*

| | |
|---|---|
| Ein Wirkstoff der Formel (I) | 5 mg |
| Magnesiumstearat | 2,0 mg |
| Milchzucker | 19,3 mg |

*6. Injektionslösung*

| | |
|---|---|
| Ein Wirkstoff der Formel (I) | 10 mg |
| Natriumchlorid | 9 mg |
| destilliertes Wasser, q.s. auf | 1,0 ml |

**Patentansprüche**

1. 10-substituierte 5-Cyanmethylen-dibenzo-[a,d]-cycloheptene der allgemeinen Formel (I)

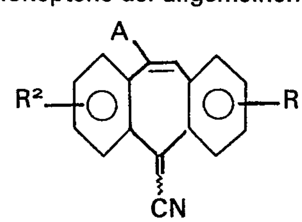

(I)

in der $R^1$ und $R^2$ Wasserstoffatome, Halogenatome, Alkylreste mit 1 bis 3 C-Atomen oder Trifluormethyl bedeuten, und A einen Aminorest $-NR^4R^5$ bedeutet, in dem $R^4$ und $R^5$ Wasserstoffatome bedeuten oder

$R^4$ und $R^5$ zusammen mit dem sie verbindenden Stickstoffatom einen 5- bis 7-gliedrigen gesättigten Ring bilden, der als weiteres Heteroatom ein Stickstoffatom enthält, wobei dieses gegebenenfalls durch einen Alkylrest mit 1 bis 3 C-Atomen oder einen Hydroxyalkylrest mit 2 bis 3 C-Atomen und gegebenenfalls zusätzlich durch Sauerstoff in Form eines N-Oxids substituiert ist,

ihre reinen cis- und trans-Isomeren und ihre physiologisch verträglichen Säureadditionssalze.

2. Verbindungen der Formel (I) nach Anspruch 1, in denen $R^1$ und $R^2$ Wasserstoff, Fluor, Chlor, Methyl oder Trifluormethyl bedeuten und A für Piperazin oder Homopiperazin steht, wobei gegebenenfalls das 4-Ringstickstoffatom durch Methyl, Äthyl, β-Hydroxyäthyl substituiert ist und gegebenenfalls in Form des N-Oxids vorliegt.

3. Verbindungen der Formel (I) nach Anspruch 1, in denen $R^1$ und $R^2$ Wasserstoff oder Chlor bedeuten und in denen für A 4-Methyl-piperazin-1-yl, 4-Äthyl-piperazin-1-yl, 4-Methyl-4-oxy-piperazin-1-yl oder N'-Methyl-homopiperazin-1-yl steht.

4. cis,trans-5-Cyanmethylen-10-(4-methylpiperazin-1-yl)-dibenzo[a,d]-cyclohepten.

5. cis-5-Cyanmethylen-10-(4-methyl-piperazin-1-yl)-dibenzo[a,d]-cyclohepten.

6. trans-5-Cyanmethylen-10-(4-methyl-piperazin-1-yl)-dibenzo[a,d]-cyclohepten.

7. cis-5-Cyanmethylen-2-chlor-10-(4-methyl-piperazin-1-yl)-dibenzo[a,d]-cyclohepten.

8. Verfahren zur Herstellung von Verbindungen der Formel (I) nach Anspruch 1, dadurch gekennzeichnet, dass man eine Verbindung der Formel (II)

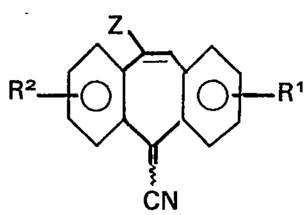

(II)

in der $R^1$ und $R^2$ die für Formel (I) angegebenen Bedeutungen haben und Z Brom oder Chlor darstellt, mit einem Nukleophil AH, in dem A die für Formel (I) angegebenen Bedeutungen hat, umsetzt, gegebenenfalls in das reine cis- und trans-Isomere trennt und gegebenenfalls die erhaltene Verbindung in das N-Oxid und/oder in das Säureadditionssalz einer physiologisch verträglichen Säure überführt.

9. Therapeutisches Mittel, gekennzeichnet durch einen Gehalt an einer Verbindung der Formel (I) nach Anspruch 1, deren reines cis- oder trans-Isomeres oder N-Oxid oder deren pharmakologisch verträgliches Säureadditionssalz als Wirkstoff neben üblichen Träger- und Verdünnungsmitteln.

10. Verbindungen nach Anspruch 1 zur Verwendung als Neuroleptikum, Sedativum, Hypnotikum oder Tranquilizer.

**Claims**

1. A 10-substituted 5-cyanomethylene-dibenzo[a,d]-cycloheptene of the general formula (I)

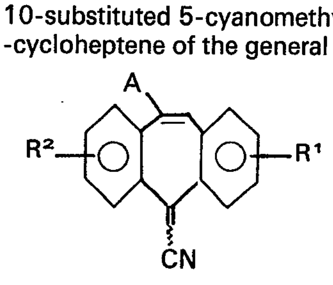

(I)

where $R^1$ and $R^2$ are hydrogen, halogen, alkyl of 1 to 3 carbon atoms or trifluoromethyl, and A is an amino radical $-NR^4R^5$, where $R^4$ and $R^5$ are each hydrogen, or $R^4$ and $R^5$ together with the nitrogen atom by which they are linked are a 5-, 6- or 7-membered saturated ring that contains a nitrogen as a further hetero-atom which is optionally substituted by alkyl of 1 to 3 carbon atoms, or hydroxyalkyl of 2 or 3 carbon atoms, and, where appropriate, is additionally substituted by oxygen in the form of an N-oxide,

its pure cis-isomer and trans-isomer, and its physiologically tolerated addition salts with acids.

2. A compound of the formula (I) as claimed in claim 1, where $R^1$ and $R^2$ are hydrogen, fluorine, chlorine, methyl or trifluoromethyl and A is piperazine or homopiperazine, in which the fourth ring nitrogen is optionally substitued by methyl, ethyl or β-hydroxyethyl, and may or may not be in the form of the N-oxide.

3. A compound of the formula (I) as claimed in claim 1, where $R^1$ and $R^2$ are hydrogen or chlorine, and A is 4-methyl-piperazin-1-yl, 4-ethyl-piperazin-1-yl, 4-methyl-4-oxypiperazin-1-yl or N'-methyl-homopiperazin-1-yl.

4. cis,trans-5-Cyanomethylene-10-(4-methyl-piperazin-1-yl)-dibenzo[a,d]-cycloheptene.

5. cis-5-Cyanomethylene-10-(4-methylpiperazin-1-yl)-dibenzo[a,d]-cycloheptene.

6. trans-5-Cyanomethylene-10-(4-methylpiperazin-1-yl)-dibenzo[a,d]-cycloheptene.

7. cis-5-Cyanomethylene-2-chloro-10-(4-methyl-piperazin-1-yl)-dibenzo[a,d]-cycloheptene.

8. A process for the preparation of a compound of the formula (I) as claimed in claim 1, wherein a compound of the formula (II)

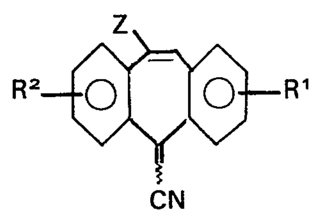

(II)

where $R^1$ and $R^2$ have the meanings given for formula (I) and Z is bromine or chlorine, is reacted with a nucleophilic agent AH, where A has the meanings given for formula (I), and, if desired, the product is separated into the pure cis-isomer and trans-isomer and, if desired, the resulting compound is converted into the N-oxide and/or into an addition salt with a physiologically tolerated acid.

9. A therapeutic agent which contains a compound of the formula (I) as claimed in claim 1, its pure cis-isomer or trans-isomer or N-oxide, or a pharmacologically tolerated acid addition salt thereof, as the active compound, in addition to conventional carriers and diluents.

10. A compound as claimed in claim 1 for use as a neuroleptic, sedative, hypnotic or tranquilizer.

## Revendications

1. 5-Cyanométhylène-dibenzo[a,d]-cyclo-heptènes substitués en position 10, de la formule générale (I)

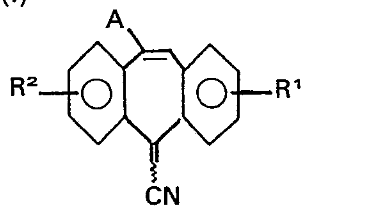

(I)

dans laquelle $R^1$ et $R^2$ désignent des atomes d'hydrogène ou d'halogène, des radicaux alkyle en $C_1$ à $C_3$ ou trifluorométhyle et A représente un groupe amino $-NR^4R^5$, où $R^4$ et $R^5$ représentent des atomes d'hydrogène ou forment avec l'atome d'azote adjacent un noyau penta- à heptagonal saturé, qui contient comme hétéro-atome additionnel un autre atome d'azote, éventuellement substitué par un radical alkyle en $C_1$ à $C_3$ ou un radical hydroxyalkyle en $C_2$ ou $C_3$ et(ou) un atome d'oxygène, formant un N-oxyde,

les isomères cis et trans purs et leurs sels d'addition d'acides physiologiquement compatibles.

2. Composés de la formule (I) selon la revendication 1, pour lesquels $R^1$ et $R^2$=H, F, Cl, méthyle ou trifluorométhyle et A représente un groupe pipérazine ou homopipérazine, l'atome d'azote en position 4 du noyau pouvant être substitué par un radical méthyle, éthyle ou β-hydroxy-éthyle ou par un atome d'oxygène, formant un N-oxyde.

3. Composés de la formule (I) selon la revendication 1, pour lesquels $R^1$ et $R^2$=H ou Cl et A représente un groupe 4-méthyl-pipérazin-1-yle, 4-éthyl-pipérazin-1-yle, 4-méthyl-4-oxy-pipérazin-1-yle ou N'-méthyl-homopipérazin-1-yle.

4. Le 5-cyanométhylène-10-(4-méthyl-pipérazin-1-yl)-dibenzo[a,d]-cycloheptène cis, trans.

5. Le 5-cyanométhylène-10-(4-méthyl-pipérazin-1-yl)-dibenzo[a,d]-cycloheptène cis.

6. Le 5-cyanométhylène-10-(4-méthyl-pipérazin-1-yl)-dibenzo[a,d]-cycloheptène trans.

7. Le 5-cyanométhylène-2-chloro-10-(4-méthyl-pipérazin-1-yl)-dibenzo[a,d]-cycloheptène cis.

8. Procédé de préparation de composés de la formule (I) selon la revendication 1, caractérisé en ce que l'on fait réagir un composé de la formule (II)

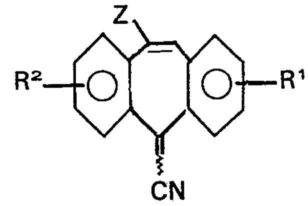

(II)

dans laquelle $R^1$ et $R^2$ possèdent les significations définies pour la formule (I) et Z désigne un atome de brome ou de chlore, avec un composé nucléophile AH, où A possède les significations définies pour la formule 1, on sépare éventuellement les isomères cis et trans purs et on transforme éventuellement le composé obtenu en N-oxyde et(ou) en un sel d'addition d'un acide physiologiquement compatible.

9. Composition thérapeutique, caractérisée en ce qu'elle contient, à côté de véhicules et diluants usuels, un composé de la formule (I) selon la revendication 1, l'isomère cis ou trans pur d'un tel composé ou son N-oxyde ou un sel d'addition d'un acide physiologiquement compatible comme principe actif.

10. Composés selon la revendication 1, destinés à être utilisés comme substances neuroleptiques, sédatives, hypnotiques ou tranquillisantes.